# EUROPEAN PATENT SPECIFICATION

(11) **EP 4 093 396 B1**
(45) Date of publication and mention of the grant of the patent: **10.12.2025**
(21) Application number: 21702886.9
(22) Date of filing: 22.01.2021
(51) Int. Cl.: A61K 9/06, A61K 31/225, A61K 31/436, A61K 31/454, A61P 17/00, A61P 17/06, A61K 9/00

(54) **TOPICAL COMPOSITIONS COMPRISING A MACROLIDE IMMUNOSUPPRESSANT**
TOPISCHE ZUSAMMENSETZUNGEN MIT EINEM MAKROLID-IMMUNSUPPRESSIVUM
COMPOSITIONS TOPIQUES COMPRENANT UN IMMUNOSUPPRESSEUR MACROLIDE

(30) Priority: 22.01.2020 US 202062964495 P; 22.01.2020 US 202062964507 P; 22.01.2020 US 202062964516 P
(43) Date of publication of application: 30.11.2022
(73) Proprietor: Bausch Health Ireland Limited, Dublin 24 (IE)
(72) Inventor: DESAI, Nayan, Irvine, California 92618 (US); CHEUNG, Yuk Ying, Irvine, California 92618 (US); PILLAI, Radhakrishnan, Irvine, California 92618 (US)
(74) Representative: Maiwald GmbH
(86) International application number: PCT/EP2021/051478
(87) International publication number: WO 2021/148619

(56) References cited:
- WO-A1-2018/102407
- US-A1- 2012 184 511
- US-A1- 2015 148 378
- US-A1- 2019 060 288
- MARSLAND A M ET AL: "Therapeutic potential of macrolide immunosuppressants in dermatology", EXPERT OPINION ON INVESTIGATIONAL DRUGS, INFORMA HEALTHCARE, UK, vol. 13, no. 2, 1 February 2004 (2004-02-01), pages 125 - 137, XP002731046, ISSN: 1354-3784, DOI: 10.1517/13543784.13.2.125
- RONAK VAKIL ET AL: "Combination Antifungal Therapy Involving Amphotericin B, Rapamycin and 5-Fluorocytosine Using PEG-Phospholipid Micelles", PHARMACEUTICAL RESEARCH, KLUWER ACADEMIC PUBLISHERS-PLENUM PUBLISHERS, NL, vol. 25, no. 9, 16 April 2008 (2008-04-16), pages 2056 - 2064, XP019613155, ISSN: 1573-904X

## Description

### FIELD

This disclosure relates to topical compositions for the treatment of inflammatory skin conditions, e.g., seborrheic dermatitis, eczema, and psoriasis. In particular, the disclosure provides topical compositions comprising a macrolide immunosuppressant (e.g., selected from pimecrolimus, sirolimus, or tacrolimus) and efinaconazole, and methods for making and using the same.

### BACKGROUND

The macrolide immunosuppressants are a class of compounds consisting of a large macrocyclic lactam/lactone ring, including natural compounds from *Streptomyces* species and derivatives thereof, which bind to immunophilins and have a range of biological effects. While these compounds have potent biological activity, they are generally poorly soluble or insoluble in water, and so present formulation challenges.

Pimecrolimus is a 33-epi-chloro derivative of ascomycin, having the structure: 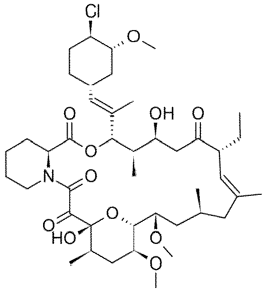 The exact mechanism of pimecrolimus is not known with certainty, but it is a calcineurin inhibitor, and appears to have immunosuppressive and anti-inflammatory effects due to its inhibition of various cytokine pathways. A 1% topical formulation of pimecrolimus (Elidel^{®}) is approved for treatment of atopic dermatitis. Pimecrolimus is soluble in methanol and ethanol, but insoluble in water. Pimecrolimus has proved difficult to formulate, due to its relative insolubility.

Sirolimus, also known as rapamycin, is a macrolide compound obtained from *Streptomyces hygroscopicus* having the structure: 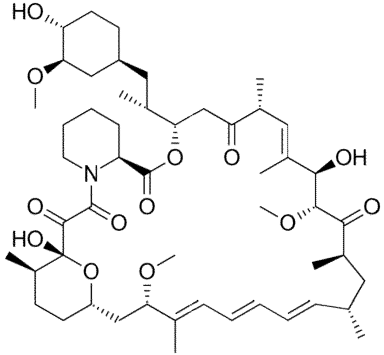 The exact mechanism of sirolimus is not known with certainty, but it has immunosuppressant, antifungal, and antineoplastic properties. It inhibits activation of T cells and B cells by reducing their sensitivity to interleukin-2 (IL-2) through mTOR inhibition. There are no topical formulations of sirolimus on the US market, but topical formulations of 0.1% or 1.0% sirolimus applied once daily have been investigated for treating facial angiofibromas, and oral tablet (1 or 2 mg) and solution (1 mg/mL) formulations have been sold under the trade name Rapamune^{®}. Sirolimus is insoluble in water, but soluble in benzyl alcohol, chloroform, acetone, and acetonitrile. Due to its aqueous insolubility, sirolimus has proved difficult to formulate.

Tacrolimus, also known as fujimycin or FK-506, is a macrolide compound obtained from *Streptomyces tsukubaensis* having the structure: 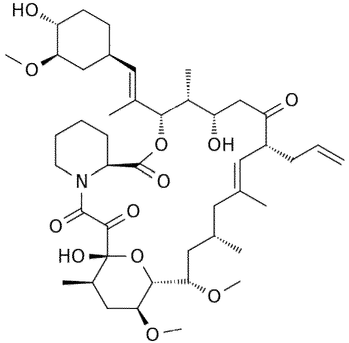 The exact mechanism of tacrolimus is not known with certainty, but it is a calcineurin inhibitor, and it appears to have immunosuppressive and anti-inflammatory effects due to its inhibition of various cytokine pathways. A topical ointment formulation of tacrolimus (Protopic^{®}) is available, having 0.03% or 0.1% of tacrolimus (w/w) in a base of mineral oil, paraffin, propylene carbonate, white petrolatum and white wax. Tacrolimus is insoluble in water, but soluble in benzyl alcohol, chloroform, acetone, and acetonitrile. Due to its aqueous insolubility, tacrolimus has proved difficult to formulate.

Efinaconazole is a triazole antifungal having the structure: 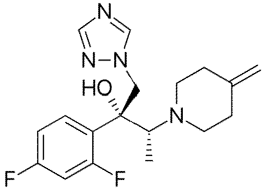 A 10% topical solution (Jublia^{®}) is approved for treatment of treatment of onychomycosis of the toenails, e.g. caused by *Trichophyton rubrum* or *Trichophyton mentagrophytes.*

There is a need for improved treatments for inflammatory conditions of the skin. Formulations which combine different active ingredients are convenient for patients but may be constrained due to unpredictable chemical interactions between the active ingredients, unpredictable effects of one active ingredient on the delivery of another active ingredient, unpredictable efficacy, and potential for unpredictable side effects. Topical formulations of macrolide immunosuppressants, particularly in the form of topical creams, may be unstable due to precipitation of the macrolide immunosuppressant. US2019060288 concerns a composition for topical application comprising: a first discontinuous phase comprising a first oil and tacrolimus; a second discontinuous phase comprising a second oil; and a continuous aqueous phase; wherein the first oil is different from the second oil. There is a need for more stable formulations of macrolide immunosuppressants, and for combination formulations in which active ingredients are selected that are safe, effective and stable in combination with one another.

### SUMMARY

The invention is defined in the appended claims.

In the present description, the references to methods of treating an inflammatory condition of the skin are to be interpreted as references to the topical composition of the present invention for use in those methods. The disclosure provides a topical formulation comprising a macrolide immunosuppressant (e.g., selected from pimecrolimus, sirolimus, or tacrolimus) and efinaconazole in an emulsion base which is unexpectedly stable and useful in the treatment of inflammatory skin conditions. The presence of efinaconazole in the emulsion results in less or slower degradation of the macrolide, while the macrolide seems to have a beneficial effect on the stability of efinaconazole.

The disclosure provides, in one embodiment, a topical pharmaceutical composition comprising a macrolide immunosuppressant and efinaconazole, for example a topical cream formulation comprising 0.01 to 1.5 wt. % of macrolide immunosuppressant and 0.5 to 3 wt.% (e.g., about 1 wt.% or about 2 wt.%) of efinaconazole. For example, the emulsion may be in the form of a cream , e.g., with a base comprising an oil phase (e.g., comprising alkyl diesters of aliphatic dicarboxylic acids), a water phase (e.g., comprising water and one or more alcohols, e.g., selected from (C₂₋₄) mono- or poly-hydric alcohols, benzyl alcohol, and combinations thereof), one or more gelling agents (e.g., comprising a carbomer), one or more surfactants (e.g., selected from anionic surfactant (e.g., selected from sodium alkyl sulfates, e,g. sodium cetostearyl sulfate), nonpolar surfactants (e.g., selected from mono- and di-glycerides), and combinations thereof), optionally an antioxidant (e.g., butylated hydroxytoluene (BHT)), and optionally a chelator (e.g., edetate disodium (EDTA)).

In another embodiment, the disclosure provides a method of treating an inflammatory condition of the skin, for example seborrheic dermatitis, eczema, or psoriasis, in a patient in need thereof, comprising administering to the affected area, e.g., once or twice daily, a combination of a macrolide immunosuppressant and efinaconazole, for example in the form of a topical cream formulation comprising 0.01 to 1.5 wt. % of the macrolide immunosuppressant and 0.5 to 3 wt.% (e.g., about 1 wt.% or about 2 wt.%) of efinaconazole, e.g., an emulsion as previously described.

For example, where the macrolide immunosuppressant is pimecrolimus, the disclosure provides a topical pharmaceutical composition comprising pimecrolimus and efinaconazole, for example a topical cream formulation comprising 0.5 to 1.5 wt. % (e.g., about 0.9 wt.%) of pimecrolimus and 0.5 to 3 wt.% (e.g., about 1 wt.% or about 2 wt.%) of efinaconazole. For example, the emulsion may be in the form of a cream , e.g., with a base comprising an oil phase (e.g., comprising alkyl diesters of aliphatic dicarboxylic acids), a water phase (e.g., comprising water and one or more alcohols, e.g., selected from (C₂₋₄) mono- or poly-hydric alcohols, benzyl alcohol, and combinations thereof), one or more gelling agents (e.g., comprising a carbomer), one or more surfactants (e.g., selected from anionic surfactant (e.g., selected from sodium alkyl sulfates, e,g. sodium cetostearyl sulfate), nonpolar surfactants (e.g., selected from mono- and di-glycerides), and combinations thereof), optionally an antioxidant (e.g., butylated hydroxytoluene (BHT)), and optionally a chelator (e.g., edetate disodium (EDTA)).

In another embodiment, the disclosure provides a method of treating an inflammatory condition of the skin, for example seborrheic dermatitis, eczema, or psoriasis, in a patient in need thereof, comprising administering to the affected area, e.g., once or twice daily, a combination of pimecrolimus and efinaconazole, for example in the form of a topical cream formulation comprising 0.5 to 1.5 wt. % (e.g., about 0.9 wt.%) of pimecrolimus and 0.5 to 3 wt.% (e.g., about 1 wt.% or about 2 wt.%) of efinaconazole, e.g., an emulsion as previously described.

Where the macrolide immunosuppressant is sirolimus, the disclosure provides a topical pharmaceutical composition comprising sirolimus and efinaconazole, for example a topical cream formulation comprising 0.1 - 1.5 wt. %, e.g., 0.5 to 1.5 wt. % (e.g., about 0.9 wt.%) of sirolimus and 0.5 to 3 wt.% (e.g., about 1 wt.% or about 2 wt.%) of efinaconazole. For example, the emulsion may be in the form of a cream , e.g., with a base comprising an oil phase (e.g., comprising alkyl diesters of aliphatic dicarboxylic acids), a water phase (e.g., comprising water and one or more alcohols, e.g., selected from (C₂₋₄) mono- or poly-hydric alcohols, benzyl alcohol, and combinations thereof), one or more gelling agents (e.g., comprising a carbomer), one or more surfactants (e.g., selected from anionic surfactant (e.g., selected from sodium alkyl sulfates, e,g. sodium cetostearyl sulfate), nonpolar surfactants (e.g., selected from mono- and diglycerides), and combinations thereof), optionally an antioxidant (e.g., butylated hydroxytoluene (BHT)), and optionally a chelator (e.g., edetate disodium (EDTA)).

In another embodiment, the disclosure provides a method of treating an inflammatory condition of the skin, for example seborrheic dermatitis, eczema, or psoriasis, in a patient in need thereof, comprising administering to the affected area, e.g., once or twice daily, a combination of sirolimus and efinaconazole, for example in the form of a topical cream formulation comprising 0.5 to 1.5 wt. % (e.g., about 0.9 wt.%) of sirolimus and 0.5 to 3 wt.% (e.g., about 1 wt.% or about 2 wt.%) of efinaconazole, e.g., an emulsion as previously described.

Where the macrolide immunosuppressant is tacrolimus, the disclosure provides a topical pharmaceutical composition comprising tacrolimus and efinaconazole, for example a topical cream formulation comprising 0.01 - 1 wt. %, e.g., about 0.03 wt % or 0.1 wt. % of tacrolimus and 0.5 to 3 wt.% (e.g., about 1 wt.% or about 2 wt.%) of efinaconazole. For example, the emulsion may be in the form of a cream , e.g., with a base comprising an oil phase (e.g., comprising alkyl diesters of aliphatic dicarboxylic acids), a water phase (e.g., comprising water and one or more alcohols, e.g., selected from (C₂₋₄) mono- or poly-hydric alcohols, benzyl alcohol, and combinations thereof), one or more gelling agents (e.g., comprising a carbomer), one or more surfactants (e.g., selected from anionic surfactant (e.g., selected from sodium alkyl sulfates, e,g. sodium cetostearyl sulfate), nonpolar surfactants (e.g., selected from mono- and di-glycerides), and combinations thereof), optionally an antioxidant (e.g., butylated hydroxytoluene (BHT)), and optionally a chelator (e.g., edetate disodium (EDTA)).

In another embodiment, the disclosure provides a method of treating an inflammatory condition of the skin, for example seborrheic dermatitis, eczema, or psoriasis, in a patient in need thereof, comprising administering to the affected area, e.g., once or twice daily, a combination of tacrolimus and efinaconazole, for example in the form of a topical cream formulation comprising 0.01 - 1 wt. %, e.g., about 0.03 wt % or 0.1 wt. % of tacrolimus and 0.5 to 3 wt.% (e.g., about 1 wt.% or about 2 wt.%) of efinaconazole, e.g., an emulsion as previously described.

Further areas of applicability of the present disclosure will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

The formulations herein are generally useful to treat inflammatory skin conditions. Inflammatory skin conditions include dermatitis (e.g., eczema (including atopic dermatitis), contact dermatitis, eczematous dermatitises, and seborrheic dermatitis), psoriasis (e.g., plaque psoriasis, guttate psoriasis, inverse psoriasis, pustular psoriasis, and erythrodermic psoriasis), lichen planus, lichen sclerosus, sclerosis, scleroderma, systemic sclerosis, hidradenitis suppurativa, pemphigus, bullous pemphigoid, epidermolysis bullosa, urticaria, angioedemas, vasculitides, erythemas (e.g., erythema multiforme, erythema nodosum), cutaneous eosinophilias, granuloma annulare, keratosis pilaris, panniculitis, pyoderma gangrenosum, Stevens-Johnson syndrome, toxic epidermal necrolysis, alopecia (e.g., alopecia areata, cicatricial alopecia), rosacea, and acne.

The formulations herein are particularly useful to treat seborrheic dermatitis, eczema or psoriasis.

In a particular embodiment, the formulations herein are useful to treat seborrheic dermatitis (also sometimes referred to as seborrheic eczema or seborrheic psoriasis, or cradle cap in infants), a common chronic inflammatory skin condition. Symptoms include scaly patches, red skin, and dandruff, usually on the scalp, but sometimes on the face or upper body. The causes of the condition are not fully understood but appear to involve interactions among the particular sebaceous gland secretions of the patient, the proliferation of *Malessezia* yeasts, and the patient's immune response.

In a first embodiment, the disclosure provides a topical pharmaceutical composition comprising a macrolide immunosuppressant and efinaconazole (Formulation A); for example,
A.1. Formulation A wherein the macrolide immunosuppressant and efinaconazole are present in concentrations effective to treat an inflammatory skin condition, e.g., seborrheic dermatitis, eczema, or psoriasis, e.g., in accordance with any of Method A, et seq., e.g., in a concentration of 0.01 - 1.5 wt.%.
A.2. Formulation A.1 wherein the macrolide immunosuppressant is selected from pimecrolimus, sirolimus, and tacrolimus.
A.3. Formulation A.2 which is a topical cream formulation comprising 0.5 to 1.5 wt. % (e.g., about 0.9 wt.%) of pimecrolimus and 0.5 to 3 wt.% of efinaconazole.
A.4. Formulation A.2 which is a topical cream formulation comprising 0.1 to 1.0 wt. % of sirolimus and 0.5 to 3 wt.% of efinaconazole.
A.5. Formulation A.2 which is a topical cream formulation comprising 0.01 to 1 wt. % of tacrolimus and 0.5 to 3 wt.% of efinaconazole.
A.6. Any foregoing formulation which is an emulsion.
A.7. Any foregoing formulation which is an emulsion comprising an oil phase, a water phase, one or more gelling agents, and one or more surfactants.
A.8. Any foregoing formulation which is an emulsion comprising an oil phase, a water phase, one or more gelling agents, and one or more surfactants, wherein the oil phase comprises alkyl diesters of aliphatic dicarboxylic acids.
A.9. Formulation A.8 wherein the alkyl diesters of aliphatic dicarboxylic acids are selected from diisopropyl adipate, diethyl sebacate, and combinations thereof.
A.10. Formulation A.8 wherein the oil phase further comprises fatty alcohols, e.g., selected from C₁₄₋₁₈ fatty alcohols, e.g, cetyl alcohol, steryl alcohol, and combinations thereof.
A.11. Formulation A.8 wherein the oil phase comprises diisopropyl adipate, diethyl sebacate, cetyl alcohol, and steryl alcohol.
A.12. Any of Formulations A.8 - A.11 wherein the oil phase comprises 10 - 60% of the formulation by weight, e.g., 40 - 50% of the formulation by weight.
A.13. Any foregoing formulation which is an emulsion comprising an oil phase, a water phase, one or more gelling agents, and one or more surfactants, wherein the water phase comprises water and one or more alcohols selected from C₂₋₄ mono- or polyhydric alcohols (e.g. propylene glycol or glycerol), benzyl alcohol, and combinations thereof.
A.14. Formulation A.13 wherein the one or more alcohols comprise propylene glycol.
A.15. Formulation A.13 or A.14 wherein the one or more alcohols comprise benzyl alcohol.
A.16. Any of Formulations A.13 - A.15 wherein the one or more alcohols comprise propylene glycol and benzyl alcohol.
A.17. Any of Formulations A.13 - A.16 wherein the one or more alcohols are present in an amount of 5-10% by weight of the formulation.
A.18. Any foregoing formulation which is an emulsion comprising an oil phase, a water phase, one or more gelling agents, and one or more surfactants, wherein the one or more gelling agents comprise a carbomer.
A.19. Formulation A.18 wherein the carbomer is a carbomer homopolymer, crosslinked with allyl ethers of polyalcohols (e.g., allyl sucrose or allyl pentaerythritol); e.g., a polymer of acrylic acid cross-linked with allyl ethers of polyalcohols; e.g., containing from 56% to 68% of carboxylic acid (-COOH) groups; e.g., having a viscosity of 40,000 - 60,000 cPs (measured at 0.5 wt% at pH 7.5).
A.20. Formulation A.18 or A.19 wherein the carbomer is a carbomer homopolymer Type C, e.g., as defined by the United States Pharmacopeia/National Formulary (USP/NF) monograph, e.g., Carbopol 980.
A.21. Formulation A.18, A.19, or A.20 wherein the gelling agent in present in an amount of 0.1-1% by weight of the formulation.
A.22. Any foregoing formulation which is an emulsion comprising an oil phase, a water phase, one or more gelling agents, and one or more surfactants, wherein the one or more surfactants are selected from anionic surfactants, nonpolar surfactants, and combinations thereof.
A.23. Formulation A.22 wherein the one or more surfactants comprise one or more or anionic surfactants selected from sodium alkyl sulfates and one or more nonpolar surfactant selected from mono- and di-glycerides and combinations thereof, e.g., wherein the glycerides are mono- and di-glycerides of fatty acids, e.g., a mixture comprising mono- and di-glycerides of lauric, linoleic, myristic, oleic, palmitic, and/or stearic acid, e.g., as defined in 21 CFR 184.1505.
A.24. Formulation A.23 wherein the one or more surfactants comprise sodium cetostearyl sulfate and mono- and di-glycerides, e.g., wherein the one or more surfactants comprise a mixture of sodium cetostearyl sulfate, glycerol monostearate, and glycerol distearate.
A.25. Any of formulations A.22, A.23, or A.24 wherein the one or more surfactants are present in an amount of 2% - 5% by weight of the formulation.
A.26. Any foregoing formulation further comprising one or more antioxidants.
A.27. Formulation 1.27 wherein the one or more antioxidants comprise butylated hydroxytoluene (BHT).
A.28. Any foregoing formulation further comprising one or more chelating agents.
A.29. Formulation A.28 wherein the one or more chelating agents are selected from citric acid, edetate disodium (EDTA) and combinations thereof.
A.30. Formulation A.29 wherein the one or more chelating agents comprise citric acid and EDTA.
A.31. Any foregoing formulation wherein the pH is 5 - 6, e.g., ca. pH 5.7.
A.32. Any foregoing formulation which is an emulsion cream comprising:
   Macrolide immunosuppressant: ca. 0.01 - 1.5%;
   Efinaconazole: ca. 1% - 2%;
   An oil phase comprising
      Diisopropyl Adipate: 15% - 25%, e.g., ca. 20%,
      Diethyl Sebacate: 20% - 25%, e.g. ca. 24%,
      Cetyl Alcohol: 1% - 2%, e.g., ca. 1.5%, and
      Stearyl Alcohol: 1% - 2%, e.g., ca. 1.5%;
   One or more surfactants comprising
      Sodium Cetostearyl Sulfate: 0.5% - 1.5%, e.g., ca. 1%, and
      Mono- and Di-glyceride: 1% - 3%, e.g., ca. 2%;
   A gelling agent comprising Carbomer Homopolymer Type C: 0.1% - 1%, e.g., ca. 0.4%;
   A water phase comprising
      Benzyl Alcohol: 0.5% - 1.5%, ca. 1%,
      Propylene Glycol: 3% - 7%, ca. 5%, and
      Water: 25% - 40%;
   One or more antioxidants comprising Butylated Hydroxytoluene (BHT): 0.05% - 0.5%, ca. 0.1%;
   One or more chelators comprising
      Citric Acid: 0.05% - 0.2%, ca. 0.1%,
      Edetate Disodium (EDTA): 0.02% - 0.1%, ca. 0.05%, and
      Sodium Hydroxide, q.s. to pH 5 - 6, e.g. ca. pH 5.7
   wherein all percentages are by weight of the formulation.
A.33. Any foregoing formulation wherein the macrolide immunosuppressant exhibits improved stability in comparison to a control formulation without efinaconazole.
A.34. Any foregoing formulation wherein the efinaconazole exhibits improved stability in comparison to a control formulation without the macrolide immunosuppressant.
A.35. Any foregoing formulation which is stable after one month of storage at 40°C.
A.36. Any foregoing formulation wherein the amount of macrolide immunosuppressant which is degraded after 3 months of storage at 40°C is less than 1% of the original amount.
A.37. Any foregoing formulation wherein the amount of macrolide immunosuppressant which is degraded after 6 months of storage at 40°C is less than 1.5% of the original amount.
A.38. Any foregoing formulation for use in treating an inflammatory skin condition.
A.39. Any foregoing formulation for use in treating seborrheic dermatitis, eczema, or psoriasis.
A.40. Any foregoing formulation for topical administration to the skin once or twice daily.
A.41. Any formulation obtained or obtainable by combination of ingredients as identified in any foregoing formulation.

In another embodiment, the disclosure provides a drug product, which is a container containing any of Formulations A, et seq., e.g., a pump container or a deformable tube containing any of Formulations A, et seq., e.g., a container comprising a pump and containing any of Formulations A, et seq., wherein the pump is calibrated to release a specific amount (e.g., 0.5-1 cubic centimeter, e.g., a pea-sized portion) of the formulation each time the pump is pressed.

In another embodiments, the disclosure provides a drug product in unit dose form (e.g., 0.5-1 cubic centimeter) comprising any of Formulations A, et seq.

In another embodiment, the disclosure provides a method (Method A) of treating inflammatory skin condition, e.g., seborrheic dermatitis, eczema, or psoriasis, in a patient in need thereof, comprising topically administering to the affected area an effective amount of macrolide immunosuppressant and efinaconazole, in combination, on at least a daily basis. For example, the disclosure provides:
A.1 Method A, wherein the macrolide immunosuppressant and efinaconazole are administered in the form of a topical cream formulation comprising 0.5% to 1.5 wt. % of sirolimus and 0.5% to 3 wt.% of efinaconazole.
A.2 Method A or A.1 wherein administration is once daily.
A.3 Any foregoing method wherein the inflammatory skin condition is selected from dermatitis (e.g., eczema (atopic dermatitis), contact dermatitis, eczematous dermatitises, and seborrheic dermatitis), psoriasis (e.g., plaque psoriasis, guttate psoriasis, inverse psoriasis, pustular psoriasis, and erythrodermic psoriasis), lichen planus, lichen sclerosus, sclerosis, scleroderma, systemic sclerosis, hidradenitis suppurativa, pemphigus, bullous pemphigoid, epidermolysis bullosa, urticaria, angioedemas, vasculitides, erythemas (e.g., erythema multiforme, erythema nodosum), cutaneous eosinophilias, granuloma annulare, keratosis pilaris, panniculitis, pyoderma gangrenosum, Stevens-Johnson syndrome, toxic epidermal necrolysis, alopecia (e.g., alopecia areata, cicatricial alopecia), rosacea, and acne.
A.4 Any foregoing method wherein the inflammatory skin condition is selected from seborrheic dermatitis, eczema and psoriasis.
A.5 Method A, A.1, A.2, A.3, or A.4, wherein the inflammatory skin condition is seborrheic dermatitis.
A.6 Method A, A.1, A.2, A.3, or A.4, wherein the inflammatory skin condition is eczema.
A.7 Method A, A.1, A.2, A.3, or A.4, wherein the inflammatory skin condition is psoriasis.
A.8 Any foregoing method wherein the affected area is the scalp, face and/or upper body.
A.9 Any foregoing method wherein the affected area is the scalp.
A.10 Any foregoing method wherein the treatment is effective to reduce or mitigate scaly patches, red skin, and/or dandruff.
A.11 Any foregoing method, wherein the step of topically administering to the affected area an effective amount of macrolide immunosuppressant and efinaconazole, in combination, comprises administering a formulation selected from any one of Formulations A - A.41.

In another aspect, the disclosure provides the use of macrolide immunosuppressant and efinaconazole, in the manufacture of a medicament (e.g., a formulation according to any one of Formulations A, et seq.) for treatment of an inflammatory condition of the skin (e.g., in accordance with any of Methods A, et seq.).

In another embodiment, the disclosure provides a combination of macrolide immunosuppressant and efinaconazole for use in the treatment of inflammatory skin condition, e.g., seborrheic dermatitis, eczema, or psoriasis, e.g., in accordance with any of Methods A, et seq.

In another embodiment, the disclosure provides the use of efinaconazole to stabilize a macrolide immunosuppressant.

In one embodiment, the disclosure provides a method of stabilizing a macrolide immunosuppressant comprising admixing the macrolide immunosuppressant with efinaconazole, for example admixing to form any of Formulations A, et seq.

As noted above, macrolide immunosuppressants have proved difficult to formulate, due to their relative insolubility. It is surprisingly found that macrolide immunosuppressants are particularly soluble and stable in alkyl diesters of aliphatic dicarboxylic acids, which can be used to provide pharmaceutical formulations having relatively high stability and reduced degradation and/or precipitation of macrolide immunosuppressant compared to prior art formulations of macrolide immunosuppressants.

In certain embodiments, the macrolide immunosuppressant is pimecrolimus. For example, the disclosure provides a topical pharmaceutical composition comprising pimecrolimus and efinaconazole (Formulation PE); for example,
PE.1 Formulation PE wherein the pimecrolimus and efinaconazole are present in concentrations effective to treat an inflammatory skin condition, e.g., seborrheic dermatitis, eczema, or psoriasis, e.g., in accordance with any of Method PE, et seq.
PE.2 Formulation PE which is a topical cream formulation comprising 0.5 to 1.5 wt. % of pimecrolimus and 0.5 to 3 wt.% of efinaconazole.
PE.3 Formulation PE wherein the concentration of pimecrolimus is about 1 wt.%.
PE.4 Formulation PE wherein the concentration of pimecrolimus is about 0.9 wt.%.
PE.5 Formulation PE, PE.1 or PE.2 wherein the concentration of efinaconazole is about 1 wt.%.
PE.6 Formulation PE, PE.1 or PE.2 wherein the concentration of efinaconazole is about 2 wt.%.
PE.7 Any foregoing formulation which is an emulsion.
PE.8 Any foregoing formulation which is an emulsion comprising an oil phase, a water phase, one or more gelling agents, and one or more surfactants.
PE.9 Any foregoing formulation which is an emulsion comprising an oil phase, a water phase, one or more gelling agents, and one or more surfactants, wherein the oil phase comprises alkyl diesters of aliphatic dicarboxylic acids.
PE.10 Formulation PE.9 wherein the alkyl diesters of aliphatic dicarboxylic acids are selected from diisopropyl adipate, diethyl sebacate, and combinations thereof.
PE.11 Any of Formulations PE.8 - PE.10 wherein the oil phase comprises fatty alcohols, e.g., selected from C₁₄₋₁₈ fatty alcohols, e.g, cetyl alcohol, steryl alcohol, and combinations thereof.
PE.12 Any of Formulations PE.8 - PE.11 wherein the oil phase comprises diisopropyl adipate, diethyl sebacate, cetyl alcohol, and steryl alcohol.
PE.13 Any of Formulations PE.8 - PE.12 wherein the oil phase comprises 10 - 60% of the formulation by weight, e.g., 40 - 50% of the formulation by weight.
PE.14 Any foregoing formulation which is an emulsion comprising an oil phase, a water phase, one or more gelling agents, and one or more surfactants, wherein the water phase comprises water and one or more alcohols selected from C₂₋₄ mono- or polyhydric alcohols (e.g. propylene glycol or glycerol), benzyl alcohol, and combinations thereof.
PE.15 Formulation PE.14 wherein the one or more alcohols comprise propylene glycol.
PE.16 Formulation PE.14 or PE.15 wherein the one or more alcohols comprise benzyl alcohol.
PE.17 Any of Formulations PE.14 - PE.16 wherein the one or more alcohols comprise propylene glycol and benzyl alcohol.
PE.18 Any of Formulations PE.14 - PE.17 wherein the one or more alcohols are present in an amount of 5-10% by weight of the formulation.
PE.19 Any foregoing formulation which is an emulsion comprising an oil phase, a water phase, one or more gelling agents, and one or more surfactants, wherein the one or more gelling agents comprise a carbomer.
PE.20 Formulation PE.19 wherein the carbomer is a carbomer homopolymer, crosslinked with allyl ethers of polyalcohols (e.g., allyl sucrose or allyl pentaerythritol); e.g., a polymer of acrylic acid cross-linked with allyl ethers of polyalcohols; e.g., containing from 56% to 68% of carboxylic acid (-COOH) groups; e.g., having a viscosity of 40,000 - 60,000 cPs (measured at 0.5 wt% at pH 7.5).
PE.21 Formulation PE.19 or PE.20 wherein the carbomer is a carbomer homopolymer Type C, e.g., as defined by the United States Pharmacopeia/National Formulary (USP/NF) monograph, e.g., Carbopol 980.
PE.22 Formulation PE.19, PE.20, or PE.21 wherein the gelling agent in present in an amount of 0.1-1% by weight of the formulation.
PE.23 Any foregoing formulation which is an emulsion comprising an oil phase, a water phase, one or more gelling agents, and one or more surfactants, wherein the one or more surfactants are selected from anionic surfactants, nonpolar surfactants, and combinations thereof.
PE.24 Formulation PE.23 wherein the one or more surfactants comprise one or more or anionic surfactants selected from sodium alkyl sulfates and one or more nonpolar surfactant selected from mono- and di-glycerides and combinations thereof, e.g., wherein the glycerides are mono- and di-glycerides of fatty acids, e.g., a mixture comprising mono- and di-glycerides of lauric, linoleic, myristic, oleic, palmitic, and/or stearic acid, e.g., as defined in 21 CFR 184.1505.
PE.25 Formulation PE.24 wherein the one or more surfactants comprise sodium cetostearyl sulfate and mono- and di-glycerides, e.g., wherein the one or more surfactants comprise a mixture of sodium cetostearyl sulfate, glycerol monostearate, and glycerol distearate
PE.26 Any of formulations PE.23, PE.24 or PE.25 wherein the one or more surfactants are present in an amount of 2% - 5% by weight of the formulation.
PE.27 Any foregoing formulation further comprising one or more antioxidants.
PE.28 Formulation PE.27 wherein the one or more antioxidants comprise butylated hydroxytoluene (BHT).
PE.29 Any foregoing formulation further comprising one or more chelating agents.
PE.30 Formulation PE.29 wherein the one or more chelating agents are selected from citric acid, edetate disodium (EDTA) and combinations thereof.
PE.31 Formulation PE.30 wherein the one or more chelating agents comprise citric acid and EDTA.
PE.32 Any foregoing formulation wherein the pH is 5 - 6, e.g., ca. pH 5.7.
PE.33 Any foregoing formulation which is an emulsion cream comprising:
   i) Pimecrolimus: ca. 0.9%
   ii) Efinaconazole: ca. 1% - 2%
   iii) An oil phase comprising
      (1) Diisopropyl Adipate: 15% - 25%, e.g., ca. 20%
      (2) Diethyl Sebacate: 20% - 25%, e.g. ca. 24%
      (3) Cetyl Alcohol: 1% - 2%, e.g., ca. 1.5%
      (4) Stearyl Alcohol: 1% - 2%, e.g., ca. 1.5%
   iv) One or more surfactants comprising
      (1) Sodium Cetostearyl Sulfate: 0.5% - 1.5%, e.g., ca. 1%
      (2) Mono- and Di-glyceride: 1% - 3%, e.g., ca. 2%
   v) A gelling agent comprising Carbomer Homopolymer Type C: 0.1% - 1%, e.g., ca. 0.4%
   vi) A water phase comprising
      (1) Benzyl Alcohol: 0.5% - 1.5%, ca. 1%
      (2) Propylene Glycol: 3% - 7%, ca. 5%
      (3) Water: 25% - 40%
   vii)One or more antioxidants comprising Butylated Hydroxytoluene (BHT): 0.05% - 0.5%, ca. 0.1%
   viii) One or more chelators comprising
      (1) Citric Acid: 0.05% - 0.2%, ca. 0.1%
      (2) Edetate Disodium (EDTA): 0.02% - 0.1%, ca. 0.05%
   ix) Sodium Hydroxide, q.s. to pH 5 - 6, e.g. ca. pH 5.7
   wherein all percentages are by weight of the formulation.
PE.34 Any foregoing formulation wherein the pimecrolimus exhibits improved stability in comparison to a control formulation without efinaconazole.
PE.35 Any foregoing formulation wherein the efinaconazole exhibits improved stability in comparison to a control formulation without the pimecrolimus.
PE.36 Any foregoing formulation which is stable after one month of storage at 40°C.
PE.37 Any foregoing formulation wherein the amount of pimecrolimus which is degraded after 3 months of storage at 40°C is less than 1% of the original amount.
PE.38 Any foregoing formulation wherein the amount of pimecrolimus which is degraded after 6 months of storage at 40°C is less than 1.5% of the original amount.
PE.39 Any foregoing formulation for use in treating an inflammatory skin condition.
PE.40 Any foregoing formulation for use in treating seborrheic dermatitis, eczema, or psoriasis.
PE.41 Any foregoing formulation for topical administration to the skin once or twice daily.
PE.42 Any formulation obtained or obtainable by combination of ingredients as identified in any foregoing formulation.

In another embodiment, the disclosure provides a drug product, which is a container containing any of Formulations PE, et seq., e.g., a pump container or a deformable tube containing any of Formulations PE, et seq., e.g., a container comprising a pump and containing any of Formulations PE, et seq., wherein the pump is calibrated to release a specific amount (e.g., 0.5-1 cubic centimeter, e.g., a pea-sized portion) of the formulation each time the pump is pressed.

In another embodiments, the disclosure provides a drug product in unit dose form (e.g., 0.5-1 cubic centimeter) comprising any of Formulations PE, et seq.

In another embodiment, the disclosure provides a method (Method PE) of treating inflammatory skin condition, e.g., seborrheic dermatitis, eczema or psoriasis, in a patient in need thereof, comprising topically administering to the affected area an effective amount of pimecrolimus and efinaconazole, in combination, on at least a daily basis. For example, the disclosure provides:
PE.1 Method PE, wherein the pimecrolimus and efinaconazole are administered in the form of a topical cream formulation comprising 0.5% to 1.5 wt. % of pimecrolimus and 0.5% to 3 wt.% of efinaconazole.
PE.2 Method PE or PE.1 wherein administration is once daily.
PE.3 Any foregoing method wherein the inflammatory skin condition is selected from dermatitis (e.g., eczema (including atopic dermatitis), contact dermatitis, eczematous dermatitises, and seborrheic dermatitis), psoriasis (e.g., plaque psoriasis, guttate psoriasis, inverse psoriasis, pustular psoriasis, and erythrodermic psoriasis), lichen planus, lichen sclerosus, sclerosis, scleroderma, systemic sclerosis, hidradenitis suppurativa, pemphigus, bullous pemphigoid, epidermolysis bullosa, urticaria, angioedemas, vasculitides, erythemas (e.g., erythema multiforme, erythema nodosum), cutaneous eosinophilias, granuloma annulare, keratosis pilaris, panniculitis, pyoderma gangrenosum, Stevens-Johnson syndrome, toxic epidermal necrolysis, alopecia (e.g., alopecia areata, cicatricial alopecia), rosacea, and acne.
PE.4 Any foregoing method wherein the inflammatory skin condition is selected from seborrheic dermatitis, eczema and psoriasis.
PE.5 Method PE, PE.1, PE.2, PE.3, or PE.4, wherein the inflammatory skin condition is seborrheic dermatitis.
PE.6 Method PE, PE.1, PE.2, PE.3, or PE.4, wherein the inflammatory skin condition is eczema.
PE.7 Method PE, PE.1, PE.2, PE.3, or PE.4, wherein the inflammatory skin condition is psoriasis.
PE.8 Any foregoing method wherein the affected area is the scalp, face and/or upper body.
PE.9 Any foregoing method wherein the affected area is the scalp.
PE.10 Any foregoing method wherein the treatment is effective to reduce or mitigate scaly patches, red skin, and/or dandruff.
PE.11 Any foregoing method, wherein the step of topically administering to the affected area an effective amount of pimecrolimus and efinaconazole, in combination, comprises administering a formulation selected from any one of Formulations PE - PE.42.

In another aspect, the disclosure provides the use of pimecrolimus and efinaconazole, in the manufacture of a medicament (e.g., a formulation according to any one of Formulations PE, et seq.) for treatment of an inflammatory condition of the skin (e.g., in accordance with any of Methods PE, et seq.).

In another embodiment, the disclosure provides a combination of pimecrolimus and efinaconazole for use in the treatment of inflammatory skin condition, e.g., seborrheic dermatitis, eczema, or psoriasis, e.g., in accordance with any of Methods PE, et seq.

In another embodiment, the disclosure provides the use of efinaconazole to stabilize pimecrolimus.

In one embodiment, the disclosure provides a method of stabilizing pimecrolimus comprising admixing the pimecrolimus with efinaconazole, for example admixing to form any of Formulations PE, et seq.

As noted above, pimecrolimus has proved difficult to formulate, due to its relative insolubility. It is surprisingly found that pimecrolimus is particularly soluble and stable in alkyl diesters of aliphatic dicarboxylic acids, which can be used to provide pharmaceutical formulations having relatively high stability and reduced degradation and/or precipitation of pimecrolimus compared to prior art formulations of pimecrolimus.

In certain embodiments, the macrolide immunosuppressant is sirolimus. For example, the disclosure provides a topical pharmaceutical composition comprising sirolimus and efinaconazole (Formulation SE); for example,
SE.1 Formulation SE wherein the sirolimus and efinaconazole are present in concentrations effective to treat an inflammatory skin condition, e.g., seborrheic dermatitis, eczema, or psoriasis, e.g., in accordance with any of Method 2, et seq.
SE.2 Formulation SE which is a topical cream formulation comprising 0.1 to 1.0 wt. % of sirolimus and 0.5 to 3 wt.% of efinaconazole.
SE.3 Formulation SE, SE.1, or SE.2 wherein the concentration of sirolimus is about 0.4 wt.%.
SE.4 Formulation SE, SE.1, or SE.2 wherein the concentration of sirolimus is about 0.2 wt.%.
SE.5 Formulation SE, SE.1, SE.2, SE.3, or SE.4 wherein the concentration of efinaconazole is about 1 wt.%.
SE.6 Formulation SE, SE.1, SE.2, SE.3, or SE.4 wherein the concentration of efinaconazole is about 2 wt.%.
SE.7 Any foregoing formulation which is an emulsion.
SE.8 Any foregoing formulation which is an emulsion comprising an oil phase, a water phase, one or more gelling agents, and one or more surfactants.
SE.9 Any foregoing formulation which is an emulsion comprising an oil phase, a water phase, one or more gelling agents, and one or more surfactants, wherein the oil phase comprises alkyl diesters of aliphatic dicarboxylic acids.
SE.10 Formulation SE.9 wherein the alkyl diesters of aliphatic dicarboxylic acids are selected from diisopropyl adipate, diethyl sebacate, and combinations thereof.
SE.11 Any of Formulations SE.8 - SE.10 wherein the oil phase comprises fatty alcohols, e.g., selected from C₁₄₋₁₈ fatty alcohols, e.g, cetyl alcohol, steryl alcohol, and combinations thereof.
SE.12 Any of Formulations SE.8 - SE.11 wherein the oil phase comprises diisopropyl adipate, diethyl sebacate, cetyl alcohol, and steryl alcohol.
SE.13 Any of Formulations SE.8 - SE.12 wherein the oil phase comprises 10 - 60% of the formulation by weight, e.g., 40 - 50% of the formulation by weight.
SE.14 Any foregoing formulation which is an emulsion comprising an oil phase, a water phase, one or more gelling agents, and one or more surfactants, wherein the water phase comprises water and one or more alcohols selected from C₂₋₄ mono- or polyhydric alcohols (e.g. propylene glycol or glycerol), benzyl alcohol, and combinations thereof.
SE.15 Formulation SE.14 wherein the one or more alcohols comprise propylene glycol.
SE.16 Formulation SE.14 or SE.15 wherein the one or more alcohols comprise benzyl alcohol.
SE.17 Any of Formulations SE.14 -SE.16 wherein the one or more alcohols comprise propylene glycol and benzyl alcohol.
SE.18 Any of Formulations SE.14 - SE.17 wherein the one or more alcohols are present in an amount of 5-10% by weight of the formulation.
SE.19 Any foregoing formulation which is an emulsion comprising an oil phase, a water phase, one or more gelling agents, and one or more surfactants, wherein the one or more gelling agents comprise a carbomer.
SE.20 Formulation SE.19 wherein the carbomer is a carbomer homopolymer, crosslinked with allyl ethers of polyalcohols (e.g., allyl sucrose or allyl pentaerythritol); e.g., a polymer of acrylic acid cross-linked with allyl ethers of polyalcohols; e.g., containing from 56% to 68% of carboxylic acid (-COOH) groups; e.g., having a viscosity of 40,000 - 60,000 cPs (measured at 0.5 wt% at pH 7.5).
SE.21 Formulation SE.19 or SE.20 wherein the carbomer is a carbomer homopolymer Type C, e.g., as defined by the United States Pharmacopeia/National Formulary (USP/NF) monograph, e.g., Carbopol 980.
SE.22 Formulation SE.19, SE.20, or SE.21 wherein the gelling agent in present in an amount of 0.1-1% by weight of the formulation.
SE.23 Any foregoing formulation which is an emulsion comprising an oil phase, a water phase, one or more gelling agents, and one or more surfactants, wherein the one or more surfactants are selected from anionic surfactants, nonpolar surfactants, and combinations thereof.
SE.24 Formulation SE.23 wherein the one or more surfactants comprise one or more or anionic surfactants selected from sodium alkyl sulfates and one or more nonpolar surfactant selected from mono- and di-glycerides and combinations thereof, e.g., wherein the glycerides are mono- and di-glycerides of fatty acids, e.g., a mixture comprising mono- and di-glycerides of lauric, linoleic, myristic, oleic, palmitic, and/or stearic acid, e.g., as defined in 21 CFR 184.1505.
SE.25 Formulation SE.24 wherein the one or more surfactants comprise sodium cetostearyl sulfate and mono- and di-glycerides, e.g., wherein the one or more surfactants comprise a mixture of sodium cetostearyl sulfate, glycerol monostearate, and glycerol distearate.
SE.26 Any of Formulations SE.23, SE.24 or SE.25 wherein the one or more surfactants are present in an amount of 2% - 5% by weight of the formulation.
SE.27 Any foregoing formulation further comprising one or more antioxidants.
SE.28 Formulation SE.27 wherein the one or more antioxidants comprise butylated hydroxytoluene (BHT).
SE.29 Any foregoing formulation further comprising one or more chelating agents.
SE.30 Formulation SE.29 wherein the one or more chelating agents are selected from citric acid, edetate disodium (EDTA) and combinations thereof.
SE.31 Formulation SE.30 wherein the one or more chelating agents comprise citric acid and EDTA.
SE.32 Any foregoing formulation wherein the pH is 5 - 6, e.g., ca. pH 5.7.
SE.33 Any foregoing formulation which is an emulsion cream comprising:
   i) Sirolimus: ca. 0.1 - 1%
   ii) Efinaconazole: ca. 1% - 2%
   iii) An oil phase comprising
      (1) Diisopropyl Adipate: 15% - 25%, e.g., ca. 20%
      (2) Diethyl Sebacate: 20% - 25%, e.g. ca. 24%
      (3) Cetyl Alcohol: 1% - 2%, e.g., ca. 1.5%
      (4) Stearyl Alcohol: 1% - 2%, e.g., ca. 1.5%
   iv) One or more surfactants comprising
      (1) Sodium Cetostearyl Sulfate: 0.5% - 1.5%, e.g., ca. 1%
      (2) Mono- and Di-glyceride: 1% - 3%, e.g., ca. 2%
   v) A gelling agent comprising Carbomer Homopolymer Type C: 0.1% - 1%, e.g., ca. 0.4%
   vi) A water phase comprising
      (1) Benzyl Alcohol: 0.5% - 1.5%, ca. 1%
      (2) Propylene Glycol: 3% - 7%, ca. 5%
      (3) Water: 25% - 40%
   vii)One or more antioxidants comprising Butylated Hydroxytoluene (BHT): 0.05% - 0.5%, ca. 0.1%
   viii) One or more chelators comprising
      (1) Citric Acid: 0.05% - 0.2%, ca. 0.1%
      (2) Edetate Disodium (EDTA): 0.02% - 0.1%, ca. 0.05%
   ix) Sodium Hydroxide, q.s. to pH 5 - 6, e.g. ca. pH 5.7
   wherein all percentages are by weight of the formulation.
SE.34 Any foregoing formulation wherein the sirolimus exhibits improved stability in comparison to a control formulation without efinaconazole.
SE.35 Any foregoing formulation wherein the efinaconazole exhibits improved stability in comparison to a control formulation without the sirolimus.
SE.36 Any foregoing formulation which is stable after one month of storage at 40°C.
SE.37 Any foregoing formulation wherein the amount of sirolimus which is degraded after 3 months of storage at 40°C is less than 1% of the original amount.
SE.38 Any foregoing formulation wherein the amount of sirolimus which is degraded after 6 months of storage at 40°C is less than 1.5% of the original amount.
SE.39 Any foregoing formulation for use in treating an inflammatory skin condition.
SE.40 Any foregoing formulation for use in treating seborrheic dermatitis, eczema, or psoriasis.
SE.41 Any foregoing formulation for topical administration to the skin once or twice daily.
SE.42 Any formulation obtained or obtainable by combination of ingredients as identified in any foregoing formulation.

In another embodiment, the disclosure provides a drug product, which is a container containing any of Formulations SE, et seq., e.g., a pump container or a deformable tube containing any of Formulations SE, et seq., e.g., a container comprising a pump and containing any of Formulations SE, et seq., wherein the pump is calibrated to release a specific amount (e.g., 0.5-1 cubic centimeter, e.g., a pea-sized portion) of the formulation each time the pump is pressed.

In another embodiments, the disclosure provides a drug product in unit dose form (e.g., 0.5-1 cubic centimeter) comprising any of Formulations SE, et seq.

In another embodiment, the disclosure provides a method (Method SE) of treating inflammatory skin condition, in a patient in need thereof, comprising topically administering to the affected area an effective amount of sirolimus and efinaconazole, in combination, on at least a daily basis. For example, the disclosure provides:
SE.1 Method SE, wherein the sirolimus and efinaconazole are administered in the form of a topical cream formulation comprising 0.5% to 1.5 wt. % of sirolimus and 0.5% to 3 wt.% of efinaconazole.
SE.2 Method SE or SE.1 wherein administration is once daily.
SE.3 Any foregoing method wherein the inflammatory skin condition is selected from dermatitis (e.g., eczema (including atopic dermatitis), contact dermatitis, eczematous dermatitises, and seborrheic dermatitis), eczema, psoriasis (e.g., plaque psoriasis, guttate psoriasis, inverse psoriasis, pustular psoriasis, and erythrodermic psoriasis), lichen planus, lichen sclerosus, sclerosis, scleroderma, systemic sclerosis, hidradenitis suppurativa, pemphigus, bullous pemphigoid, epidermolysis bullosa, urticaria, angioedemas, vasculitides, erythemas (e.g., erythema multiforme, erythema nodosum), cutaneous eosinophilias, granuloma annulare, keratosis pilaris, panniculitis, pyoderma gangrenosum, Stevens-Johnson syndrome, toxic epidermal necrolysis, alopecia (e.g., alopecia areata, cicatricial alopecia), rosacea, and acne.
SE.4 Any foregoing method wherein the inflammatory skin condition is selected from seborrheic dermatitis, eczema and psoriasis.
SE.5 Method SE, SE.1, SE.2, SE.3, or SE.4, wherein the inflammatory skin condition is seborrheic dermatitis.
SE.6 Method SE, SE.1, SE.2, SE.3, or SE.4, wherein the inflammatory skin condition is eczema.
SE.7 Method SE, SE.1, SE.2, SE.3, or SE.4, wherein the inflammatory skin condition is psoriasis.
SE.8 Any foregoing method wherein the affected area is the scalp, face and/or upper body.
SE.9 Any foregoing method wherein the affected area is the scalp.
SE.10 Any foregoing method wherein the treatment is effective to reduce or mitigate scaly patches, red skin, and/or dandruff.
SE.11 Any foregoing method, wherein the step of topically administering to the affected area an effective amount of sirolimus and efinaconazole, in combination, comprises administering a formulation selected from any one of Formulations SE - SE.42.

In another aspect, the disclosure provides the use of sirolimus and efinaconazole, in the manufacture of a medicament (e.g., a formulation according to any one of Formulations SE, et seq.) for treatment of an inflammatory condition of the skin (e.g., in accordance with any of Methods SE, et seq.).

In another embodiment, the disclosure provides a combination of sirolimus and efinaconazole for use in the treatment of inflammatory skin condition, e.g., seborrheic dermatitis, eczema, or psoriasis, e.g., in accordance with any of Methods SE, et seq.

In another embodiment, the disclosure provides the use of efinaconazole to stabilize sirolimus.

In one embodiment, the disclosure provides a method of stabilizing sirolimus comprising admixing the sirolimus with efinaconazole, for example admixing to form any of Formulations SE, et seq.

As noted above, sirolimus has proved difficult to formulate, due to its relative insolubility. It is surprisingly found that sirolimus is particularly soluble and stable in alkyl diesters of aliphatic dicarboxylic acids, which can be used to provide pharmaceutical formulations having relatively high stability and reduced degradation and/or precipitation of sirolimus compared to prior art formulations of sirolimus.

In certain embodiments, the macrolide immunosuppressant is tacrolimus. For example, the disclosure provides a topical pharmaceutical composition comprising tacrolimus and efinaconazole (Formulation TE); for example,
TE.1 Formulation TE wherein the tacrolimus and efinaconazole are present in concentrations effective to treat an inflammatory skin condition, e.g., seborrheic dermatitis, eczema, or psoriasis, e.g., in accordance with any of Method TE, et seq.
TE.2 Formulation TE which is a topical cream formulation comprising 0.01 - 1 wt. %, e.g., 0.01 - 0.1%, of tacrolimus, and 0.5 to 3 wt.% of efinaconazole.
TE.3 Formulation TE, TE.1, or TE.2 wherein the concentration of tacrolimus is about 0.03 wt %.
TE.4 Formulation TE, TE.1, or TE.2 wherein the concentration of tacrolimus is about 0.1 wt.%.
TE.5 Formulation TE, TE.1, TE.2, TE.3, or TE.4 wherein the concentration of efinaconazole is about 1 wt.%.
TE.6 Formulation TE, TE.1, TE.2, TE.3, or TE.4 wherein the concentration of efinaconazole is about 2 wt.%.
TE.7 Any foregoing formulation which is an emulsion.
TE.8 Any foregoing formulation which is an emulsion comprising an oil phase, a water phase, one or more gelling agents, and one or more surfactants.
TE.9 Any foregoing formulation which is an emulsion comprising an oil phase, a water phase, one or more gelling agents, and one or more surfactants, wherein the oil phase comprises alkyl diesters of aliphatic dicarboxylic acids.
TE.10 Formulation TE.9 wherein the alkyl diesters of aliphatic dicarboxylic acids are selected from diisopropyl adipate, diethyl sebacate, and combinations thereof.
TE.11 Any of Formulation TE.8 - TE.10 wherein the oil phase comprises fatty alcohols, e.g., selected from C₁₄₋₁₈ fatty alcohols, e.g, cetyl alcohol, steryl alcohol, and combinations thereof.
TE.12 Any of Formulation TE.8 - TE.11 wherein the oil phase comprises diisopropyl adipate, diethyl sebacate, cetyl alcohol, and steryl alcohol.
TE.13 Any of Formulations TE.8 - TE.12 wherein the oil phase comprises 10 - 60% of the formulation by weight, e.g., 40 - 50% of the formulation by weight.
TE.14 Any foregoing formulation which is an emulsion comprising an oil phase, a water phase, one or more gelling agents, and one or more surfactants, wherein the water phase comprises water and one or more alcohols selected from C₂₋₄ mono- or polyhydric alcohols (e.g. propylene glycol or glycerol), benzyl alcohol, and combinations thereof.
TE.15 Formulation TE.14 wherein the one or more alcohols comprise propylene glycol.
TE.16 Formulation TE.14 or TE.15 wherein the one or more alcohols comprise benzyl alcohol.
TE.17 Any of Formulations TE.14 - TE.16 wherein the one or more alcohols comprise propylene glycol and benzyl alcohol.
TE.18 Any of Formulations TE.14 - TE.17 wherein the one or more alcohols are present in an amount of 5-10% by weight of the formulation.
TE.19 Any foregoing formulation which is an emulsion comprising an oil phase, a water phase, one or more gelling agents, and one or more surfactants, wherein the one or more gelling agents comprise a carbomer.
TE.20 Formulation TE.19 wherein the carbomer is a carbomer homopolymer, crosslinked with allyl ethers of polyalcohols (e.g., allyl sucrose or allyl pentaerythritol); e.g., a polymer of acrylic acid cross-linked with allyl ethers of polyalcohols; e.g., containing from 56% to 68% of carboxylic acid (-COOH) groups; e.g., having a viscosity of 40,000 - 60,000 cPs (measured at 0.5 wt% at pH 7.5).
TE.21 Formulation TE.19 or TE.20 wherein the carbomer is a carbomer homopolymer Type C, e.g., as defined by the United States Pharmacopeia/National Formulary (USP/NF) monograph, e.g., Carbopol 980.
TE.22 Formulation TE.19, TE.20, or TE.21 wherein the one or more gelling agents are present in an amount of 0.1-1% by weight of the formulation.
TE.23 Any foregoing formulation which is an emulsion comprising an oil phase, a water phase, one or more gelling agents, and one or more surfactants, wherein the one or more surfactants are selected from anionic surfactants, nonpolar surfactants, and combinations thereof.
TE.24 Formulation TE.23 wherein the one or more surfactants comprise one or more or anionic surfactants selected from sodium alkyl sulfates and one or more nonpolar surfactant selected from mono- and di-glycerides and combinations thereof, e.g., wherein the glycerides are mono- and di-glycerides of fatty acids, e.g., a mixture comprising mono- and di-glycerides of lauric, linoleic, myristic, oleic, palmitic, and/or stearic acid, e.g., as defined in 21 CFR 184.1505.
TE.25 Formulation TE.23 or TE.24 wherein the one or more surfactants comprise sodium cetostearyl sulfate and mono- and di-glycerides, e.g., wherein the one or more surfactants comprise a mixture of sodium cetostearyl sulfate, glycerol monostearate, and glycerol distearate
TE.26 Any of Formulations TE.23 - TE.25 wherein the one or more surfactants are present in an amount of 2% - 5% by weight of the formulation.
TE.27 Any foregoing formulation further comprising one or more antioxidants.
TE.28 Formulation TE.27 wherein the one or more antioxidants comprise butylated hydroxytoluene (BHT).
TE.29 Any foregoing formulation further comprising one or more chelating agents.
TE.30 Formulation TE.29 wherein the one or more chelating agents are selected from citric acid, edetate disodium (EDTA) and combinations thereof.
TE.31 Formulation TE.30 wherein the one or more chelating agents comprise citric acid and EDTA.
TE.32 Any foregoing formulation wherein the pH is 5 - 6, e.g., ca. pH 5.7.
TE.33 Any foregoing formulation which is an emulsion cream comprising:
   i) Tacrolimus: ca. 0.01 - 0.1%
   ii) Efinaconazole: ca. 1% - 2%
   iii) An oil phase comprising
      (1) Diisopropyl Adipate: 15% - 25%, e.g., ca. 20%
      (2) Diethyl Sebacate: 20% - 25%, e.g. ca. 24%
      (3) Cetyl Alcohol: 1% - 2%, e.g., ca. 1.5%
      (4) Stearyl Alcohol: 1% - 2%, e.g., ca. 1.5%
   iv) One or more surfactants comprising
      (1) Sodium Cetostearyl Sulfate: 0.5% - 1.5%, e.g., ca. 1%
      (2) Mono- and Di-glyceride: 1% - 3%, e.g., ca. 2%
   v) A gelling agent comprising Carbomer Homopolymer Type C: 0.1% - 1%, e.g., ca. 0.4%
   vi) A water phase comprising
      (1) Benzyl Alcohol: 0.5% - 1.5%, ca. 1%
      (2) Propylene Glycol: 3% - 7%, ca. 5%
      (3) Water: 25% - 40%
   vii)One or more antioxidants comprising Butylated Hydroxytoluene (BHT): 0.05% - 0.5%, ca. 0.1%
   viii) One or more chelators comprising
      (1) Citric Acid: 0.05% - 0.2%, ca. 0.1%
      (2) Edetate Disodium (EDTA): 0.02% - 0.1%, ca. 0.05%
   ix) Sodium Hydroxide, q.s. to pH 5 - 6, e.g. ca. pH 5.7
   wherein all percentages are by weight of the formulation.
TE.34 Any foregoing formulation wherein the tacrolimus exhibits improved stability in comparison to a control formulation without efinaconazole.
TE.35 Any foregoing formulation wherein the efinaconazole exhibits improved stability in comparison to a control formulation without the tacrolimus.
TE.36 Any foregoing formulation which is stable after one month of storage at 40°C.
TE.37 Any foregoing formulation wherein the amount of tacrolimus which is degraded after 3 months of storage at 40°C is less than 1% of the original amount.
TE.38 Any foregoing formulation wherein the amount of tacrolimus which is degraded after 6 months of storage at 40°C is less than 1.5% of the original amount.
TE.39 Any foregoing formulation for use in treating an inflammatory skin condition.
TE.40 Any foregoing formulation for use in treating seborrheic dermatitis, eczema, or psoriasis.
TE.41 Any foregoing formulation for topical administration to the skin once or twice daily.
TE.42 Any formulation obtained or obtainable by combination of ingredients as identified in any foregoing formulation.

In another embodiment, the disclosure provides a drug product, which is a container containing any of Formulations TE, et seq., e.g., a pump container or a deformable tube containing any of Formulations TE, et seq., e.g., a container comprising a pump and containing any of Formulations TE, et seq., wherein the pump is calibrated to release a specific amount (e.g., 0.5-1 cubic centimeter, e.g., a pea-sized portion) of the formulation each time the pump is pressed.

In another embodiment, the disclosure provides a drug product in unit dose form (e.g., 0.5-1 cubic centimeter) comprising any of Formulations TE, et seq.

In another embodiment, the disclosure provides a method (Method TE) of treating inflammatory skin condition, e.g., seborrheic dermatitis, eczema, or psoriasis, in a patient in need thereof, comprising topically administering to the affected area an effective amount of tacrolimus and efinaconazole, in combination, on at least a daily basis. For example, the disclosure provides:
TE.1 Method TE, wherein the tacrolimus and efinaconazole are administered in the form of a topical cream formulation comprising 0.01% to 1 wt. %, e.g., 0.01 - 0.1%, of tacrolimus and 0.5% to 3 wt.% of efinaconazole.
TE.2 Method TE or TE.1 wherein administration is once daily.
TE.3 Any foregoing method wherein the inflammatory skin condition is selected from dermatitis (e.g., eczema (including atopic dermatitis), contact dermatitis, eczematous dermatitises, and seborrheic dermatitis), eczema, psoriasis (e.g., plaque psoriasis, guttate psoriasis, inverse psoriasis, pustular psoriasis, and erythrodermic psoriasis), lichen planus, lichen sclerosus, sclerosis, scleroderma, systemic sclerosis, hidradenitis suppurativa, pemphigus, bullous pemphigoid, epidermolysis bullosa, urticaria, angioedemas, vasculitides, erythemas (e.g., erythema multiforme, erythema nodosum), cutaneous eosinophilias, granuloma annulare, keratosis pilaris, panniculitis, pyoderma gangrenosum, Stevens-Johnson syndrome, toxic epidermal necrolysis, alopecia (e.g., alopecia areata, cicatricial alopecia), rosacea, and acne.
TE.4 Any foregoing method wherein the inflammatory skin condition is selected from seborrheic dermatitis, eczema and psoriasis.
TE.5 Method TE, TE.1, TE.2, TE.3, or TE.4, wherein the inflammatory skin condition is seborrheic dermatitis.
TE.6 Method TE, TE.1, TE.2, TE.3, or TE.4, wherein the inflammatory skin condition is eczema.
TE.7 Method TE, TE.1, TE.2, TE.3, or TE.4, wherein the inflammatory skin condition is psoriasis.
TE.8 Method TE, TE.1, TE.2, TE.3, or TE.4, wherein the inflammatory skin condition is seborrheic dermatitis.
TE.9 Any foregoing method wherein the affected area is the scalp, face and/or upper body.
TE.10 Any foregoing method wherein the affected area is the scalp.
TE.11 Any foregoing method wherein the treatment is effective to reduce or mitigate scaly patches, red skin, and/or dandruff.
TE.12 Any foregoing method, wherein the step of topically administering to the affected area an effective amount of tacrolimus and efinaconazole, in combination, comprises administering a formulation selected from any one of Formulations TE - TE.42.

In another aspect, the disclosure provides the use of tacrolimus and efinaconazole, in the manufacture of a medicament (e.g., a formulation according to any one of Formulations TE, et seq.) for treatment of an inflammatory condition of the skin (e.g., in accordance with any of Methods TE, et seq.).

In another embodiment, the disclosure provides a combination of tacrolimus and efinaconazole for use in the treatment of inflammatory skin condition, e.g., seborrheic dermatitis, eczema, or psoriasis, e.g., in accordance with any of Methods TE, et seq.

In another embodiment, the disclosure provides the use of efinaconazole to stabilize tacrolimus.

In one embodiment, the disclosure provides a method of stabilizing tacrolimus comprising admixing the tacrolimus with efinaconazole, for example admixing to form any of Formulations TE, et seq.

As noted above, tacrolimus has proved difficult to formulate, due to its relative insolubility. It is surprisingly found that tacrolimus is particularly soluble and stable in alkyl diesters of aliphatic dicarboxylic acids, which can be used to provide pharmaceutical formulations having relatively high stability and reduced degradation and/or precipitation of tacrolimus compared to prior art formulations of tacrolimus.

Unless stated otherwise, all percentages of composition components given in this specification are by weight based on a total composition or formulation weight of 100%.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

"About" with respect to an amount or a concentration means 80% to 120%, e.g., 90% to 110%, for example 95% - 105% or +/-5% of the claimed value.

It will be understood that in certain formulations a particular ingredient may have more than one function. For example, relatively lipophilic nonpolar surfactants such as glycerol monostearate or glycerol distearate may form all or part of the oil phase of an emulsion formulation, as well as helping to keep the oil phase emulsified and stable.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The invention is further illustrated in the following examples, which are meant to be exemplary and not limiting.

### EXAMPLES

### Example 1: Exemplary formulations - Mutually beneficial effect of pimecrolimus and efinaconazole on stability

Three variations of a topical cream are manufactured at similar scale (500 to 1000g), packaged in the same aluminum tube and placed on stability. Compositions of the 3 creams are provided in Table 1. The only difference in the three is the concentration of efinaconazole, which ranges from 0 to 2% w/w. A comparison of the total pimecrolimus -related substances in the 3 formulations at one month is shown in Table 2.

**Table 1: Quantitative Composition of Pimecrolimus and Efinaconazole Creams**

| Component | Concentration (% w/w) | | |
|---|---|---|---|
| | Formula A | Formula B | Formula C |
| Pimecrolimus | 0.9 | 0.9 | 0.9 |
| Efinaconazole | 0 | 1.0 | 2.0 |
| Diisopropyl Adipate | 20.0 | 20.0 | 20.0 |
| Diethyl Sebacate | 24.0 | 24.0 | 24.0 |
| Sodium Cetostearyl Sulfate | 1.0 | 1.0 | 1.0 |
| Mono- and Di-glyceride | 2.0 | 2.0 | 2.0 |
| Carbopol 980 (Carbomer Homopolymer Type C) | 0.4 | 0.4 | 0.4 |
| Cetyl Alcohol | 1.5 | 1.5 | 1.5 |
| Stearyl Alcohol | 1.5 | 1.5 | 1.5 |
| Benzyl Alcohol | 1.0 | 1.0 | 1.0 |
| Propylene Glycol | 5.0 | 5.0 | 5.0 |
| Butylated Hydroxytoluene (BHT) | 0.1 | 0.1 | 3.1 |
| Citric Acid, Anhydrous | 0.1 | 0.1 | 0.1 |
| Edetate Disodium (EDTA) | 0.05 | 0.05 | 3.05 |
| Sodium Hydroxide | qs pH (5.7) | qs pH (5.7) | qs pH (5.7) |
| Purified water | qs to 100% | qs to 100% | qs to 100% |

**Table 2: Comparison of Pimecrolimus-related Substances - Effect of Efinaconazole**

| | Formula A | Formula B | Formula C |
|---|---|---|---|
| Pimecrolimus , %w/w | 0.9 | 0.9 | 0.9 |
| Efinaconazole, % w/w | 0 | 1.0 | 2.0 |
| *Total pimecrolimus related substances (% of original 0.9% w*/*w)* | | | |
| at t=0 | <0.1 | <0.1 | <0.1 |
| At 1month @ RT | <0.1 | <0.1 | <0.1 |
| At 1 month @ 40°C | 0.16 | <0.1 | <0.1 |

After one month at 40°C, no measurable degradants are noted in the creams containing both efinaconazole and pimecrolimus (Formulas B and C). On the other hand, Formula A (pimecrolimus alone, no efinaconazole) already exhibits 0.16% of total related substances.

A further experiment using larger batches of the three formulations of Table 1, studied for a longer period of time, further supports these results. Large size (35 Kg) batches of the 3 formulas in Table 1 are evaluated for stability under various storage conditions (three months at temperature up to 40°C, and relative humidity (RH) up to 75%). The lots are all manufactured at the same scale utilizing the same process and tested at about the same time using validated chemical analysis methods. Three-month data are now available and provided below in a chart and a table. These data confirm the conclusions from the earlier results - that the presence of efinaconazole stabilizes pimecrolimus in this base.

**Table 3: Beneficial Effect of Efinaconazole on Pimecrolimus-Related Impurities (% of original 0.9% w/w)**

| Storage Conditions | Storage Time | Formula A (0.9% pimecrolimus alone) | Formula B (0.9% pimecrolimus + 1% efinaconazole) | Formula C (0.9% pimecrolimus + 2% efinaconazole) |
|---|---|---|---|---|
| 25°C | T=0 | 0.12 | 0.10 | 0.10 |
| 25°C/60% RH | T=3 months | 0.34 | 0.10 | 0.12 |
| 30°C/65% RH | T=3 months | 0.42 | 0.11 | 0.14 |
| 40°C/75% RH | T=3 months | 1.10 | 0.46 | 0.46 |

These results suggest mutually beneficial behavior of the two drug substances. In each case, chemical stability of pimecrolimus, as measured by total related substances, appears to be enhanced by the presence of efinaconazole.

### Example 2: Comparison of combination lotion to pimecrolimus-only lotion

The formula composition of the two products is provided in Table 3. Other than the difference in the drug substances (monad vs combination), the main difference is the composition of the oil phase solvents.

**Table 4: Quantitative Compositions of Pimecrolimus Lotion and Combination Product**

| **Component** | **Concentration (% w/w)** | |
|---|---|---|
| | Pimecrolimus Lotion | Formula C |
| Pimecrolimus | 1.0 | 0.9 |
| Efinaconazole | 0 | 2.0 |
| Caprylic/Capric Triglycerides | 15 | 0 |
| Oleyl Alcohol | 10 | 0 |
| Diisopropyl Adipate | 0 | 20.0 |
| Diethyl Sebacate | 0 | 24.0 |
| Sodium Cetostearyl Sulfate | 1.0 | 1.0 |
| Mono- and Di-glyceride | 2.0 | 2.0 |
| Carbopol 981 (Carbomer Homopolymer Type A) | 0.2 | 0 |
| Carbopol 980 (Carbomer Homopolymer Type C) | 0 | 0.4 |
| Cetyl Alcohol | 1.0 | 1.5 |
| Stearyl Alcohol | 1.0 | 1.5 |
| Benzyl Alcohol | 1.0 | 1.0 |
| Propylene Glycol | 5.0 | 5.0 |
| Butylated Hydroxytoluene (BHT) | 0 | 0.1 |
| Citric Acid, Anhydrous | 0.05 | 0.1 |
| Edetate Disodium (EDTA) | 0 | 0.05 |
| Sodium Hydroxide | qs pH (5.7) | qs pH (5.7) |
| Purified water | qs to 100% | qs to 100% |

Test results of the total pimecrolimus -related substances are shown in Table 5. Six months of data at 40°C are available and provided in the table. Difference in the total related substances between the two formulas is substantial at every time point. As before, the presence of the other drug substance - efinaconazole - appears to have significantly suppressed the degradation of pimecrolimus.

**Table 5: Total pimecrolimus-related substances in Pimecrolimus Lotion compared to Formula C**

| | Pimecrolimus Lotion | Formula C |
|---|---|---|
| Pimecrolimus, % w/w | 1.0 | 0.9 |
| Efinaconazole, % w/w | 0 | 2.0 |
| *Total pimecrolimus-related substances* | | |
| T=0 | 0.23 | < 0.1 |
| T=1 month | 1.0 | <0.1 |
| T= 3 months | 1.2 | 0.57 |
| T=6 months | 2.5 | 1.0 |

Moreover, comparing the stability of the pimecrolimus-only Formula A of Table 3 with the stability of the lotion in Table 5 suggests that Formula A shows superior stability to the pimecrolimus lotion, although it is not a side-by-side comparison.

*Comparison of Pimecrolimus-only lotion and Pimecrolimus-Efinaconazole combination product at pH 5:* Pimecrolimus in the above lotion base is discovered to be more susceptible to degradation at lower pH (around 5), compared to the combination product of Formula C, as seen in the level of total pimecrolimus-related substances shown in Table 6. The total related substances value is 2.2 % of the original concentration after 3 months at 40°C. By comparison, the same value in the cream containing the combination of the drug substances is 0.74%-- an improvement of 65% over the pimecrolimus-only product.

**Table 6: Comparison of Pimecrolimus-only Lotion with Combination Product from Table 3, but adjusted to pH 5 instead of pH 5.7**

| | Pimecrolimus Lotion (pH 5) | Formula C (pH 5) |
|---|---|---|
| Pimecrolimus, %w/w | 1.0 | 0.9 |
| Efinaconazole, %w/w | 0 | 2.0 |

| Total pimecrolimus-related substances | | |
|---|---|---|
| T=0 | 0.2 | < 0.1 |
| T=1 month | 0.86 | <0.1 |
| T= 3 months | 2.2 | 0.74 |

### Example 3: Effect of Pimecrolimus on the stability of Efinaconazole

Data presented in the previous Examples support the hypotheses that the presence of efinaconazole results in less degradation of pimecrolimus in emulsions. It appears that the converse is also true, *i.e.* the presence of pimecrolimus aids in retarding the degradation of efinaconazole, as shown in Table 6. While the efinaconazole-only formulation is an alcohol-based solution and the combination formulation (Formula C from Example 1, *supra*) is an oil and water cream, the total efinaconazole-related substances are measurably lower in the combination product than in the efinaconazole-only product.

**Table 6: Efinaconazole-related substances in combination product and monad**

| | Efinaconazole-only product | Formula C |
|---|---|---|
| Pimecrolimus, % w/w | 0 | 0.9 |
| Efinaconazole , % w/w | 10 | 2.0 |

| Total efinaconazole -related substances | | |
|---|---|---|
| T=0 | NRP | NRP |
| T=1 month | NRP | NRP |
| T= 3 months | 0.19 | NRP |
| T=6 months | 0.35 | 0.10 |

These test data suggest that when present together, pimecrolimus and efinaconazole act in a mutually beneficial manner, each helping in retarding the degradation of the other drug substance.

### Example 4: Exemplary formulations - Mutually beneficial effect of sirolimus and efinaconazole on stability

Three variations of a topical cream are manufactured at similar scale (500 to 1000g), packaged in the same aluminum tube and placed on stability. Compositions of the 3 creams are provided in Table 7. The only difference in the three is the concentration of efinaconazole, which ranges from 0 to 2% w/w.

**Table 7: Quantitative Composition of Sirolimus and Efinaconazole Creams**

| Component | Concentration (% w/w) | | |
|---|---|---|---|
| | Formula A | Formula B | Formula C |
| Sirolimus | 0.2 | 0.2 | 0.2 |
| Efinaconazole | 0 | 1.0 | 2.0 |
| Diisopropyl Adipate | 20.0 | 20.0 | 20.0 |
| Diethyl Sebacate | 24.0 | 24.0 | 24.0 |
| Sodium Cetostearyl Sulfate | 1.0 | 1.0 | 1.0 |
| Mono- and Di-glyceride | 2.0 | 2.0 | 2.0 |
| Carbopol 980 (Carbomer Homopolymer Type C) | 0.4 | 0.4 | 0.4 |
| Cetyl Alcohol | 1.5 | 1.5 | 1.5 |
| Stearyl Alcohol | 1.5 | 1.5 | 1.5 |
| Benzyl Alcohol | 1.0 | 1.0 | 1.0 |
| Propylene Glycol | 5.0 | 5.0 | 5.0 |
| Butylated Hydroxytoluene (BHT) | 0.1 | 0.1 | 0.1 |
| Citric Acid, Anhydrous | 0.1 | 0.1 | 0.1 |
| Edetate Disodium (EDTA) | 0.05 | 0.05 | 0.05 |
| Sodium Hydroxide | qs pH (5.7) | qs pH (5.7) | qs pH (5.7) |
| Purified water | qs to 100% | qs to 100% | qs to 100% |

After one month at 40°C, no measurable degradants are noted in the creams containing both efinaconazole and sirolimus (Formulas B and C). On the other hand, Formula A (sirolimus alone, no efinaconazole) already exhibits formation of products of sirolimus degradation.

A further experiment using larger batches of the three formulations of Table 7 is conducted. Large size (35 Kg) batches of the 3 formulas in Table 7 are evaluated for stability under various storage conditions (three months at temperature up to 40°C, and relative humidity (RH) up to 75%). The lots are all manufactured at the same scale utilizing the same process and tested at about the same time using validated chemical analysis methods. Three-month data show that the presence of efinaconazole stabilizes sirolimus in this base.

### Example 5: Comparison of combination lotion to sirolimus-only lotion

The formula composition of the two products is provided in Table 8. Other than the difference in the drug substances (monad vs combination), the main difference is the composition of the oil phase solvents.

**Table 8: Quantitative Compositions of Sirolimus Lotion and Combination Product**

| **Component** | **Concentration (% w/w)** | |
|---|---|---|
| | Sirolimus Lotion | Formula C |
| Sirolimus | 0.2 | 0.2 |
| Efinaconazole | 0 | 2.0 |
| Caprylic/Capric Triglycerides | 15 | 0 |
| Oleyl Alcohol | 10 | 0 |
| Diisopropyl Adipate | 0 | 20.0 |
| Diethyl Sebacate | 0 | 24.0 |
| Sodium Cetostearyl Sulfate | 1.0 | 1.0 |
| Mono- and Di-glyceride | 2.0 | 2.0 |
| Carbopol 981 (Carbomer Homopolymer Type A) | 0.2 | 0 |
| Carbopol 980 (Carbomer Homopolymer Type C) | 0 | 0.4 |
| Cetyl Alcohol | 1.0 | 1.5 |
| Stearyl Alcohol | 1.0 | 1.5 |
| Benzyl Alcohol | 1.0 | 1.0 |
| Propylene Glycol | 5.0 | 5.0 |
| Butylated Hydroxytoluene (BHT) | 0 | 0.1 |
| Citric Acid, Anhydrous | 0.05 | 0.1 |
| Edetate Disodium (EDTA) | 0 | 0.05 |
| Sodium Hydroxide | qs pH (5.7) | qs pH (5.7) |
| Purified water | qs to 100% | qs to 100% |

It is believed that sirolimus and efinaconazole act in a mutually beneficial manner, each helping in retarding the degradation of the other drug substance.

### Example 6: Exemplary formulations - Mutually beneficial effect of tacrolimus and efinaconazole on stability

Three variations of a topical cream are manufactured at similar scale (500 to 1000g), packaged in the same aluminum tube and placed on stability. Compositions of the 3 creams are provided in Table 9. The only difference in the three is the concentration of efinaconazole, which ranges from 0 to 2% w/w.

**Table 9: Quantitative Composition of Tacrolimus and Efinaconazole Creams**

| Component | Concentration (% w/w) | | |
|---|---|---|---|
| | Formula A | Formula B | Formula C |
| Tacrolimus | 0.1 | 0.1 | 0.1 |
| Efinaconazole | 0 | 1.0 | 2.0 |
| Diisopropyl Adipate | 20.0 | 20.0 | 20.0 |
| Diethyl Sebacate | 24.0 | 24.0 | 24.0 |
| Sodium Cetostearyl Sulfate | 1.0 | 1.0 | 1.0 |
| Mono- and Di-glyceride | 2.0 | 2.0 | 2.0 |
| Carbopol 980 (Carbomer Homopolymer Type C) | 0.4 | 0.4 | 0.4 |
| Cetyl Alcohol | 1.5 | 1.5 | 1.5 |
| Stearyl Alcohol | 1.5 | 1.5 | 1.5 |
| Benzyl Alcohol | 1.0 | 1.0 | 1.0 |
| Propylene Glycol | 5.0 | 5.0 | 5.0 |
| Butylated Hydroxytoluene (BHT) | 0.1 | 0.1 | 0.1 |
| Citric Acid, Anhydrous | 0.1 | 0.1 | 0.1 |
| Edetate Disodium (EDTA) | 0.05 | 0.05 | 3.05 |
| Sodium Hydroxide | qs pH (5.7) | qs pH (5.7) | qs pH (5.7) |
| Purified water | qs to 100% | qs to 100% | qs to 100% |

After one month at 40°C, no measurable degradants are noted in the creams containing both efinaconazole and tacrolimus (Formulas B and C). On the other hand, Formula A (tacrolimus alone, no efinaconazole) already exhibits formation of products of tacrolimus degradation.

A further experiment using larger batches of the three formulations of Table 1 is conducted. Large size (35 Kg) batches of the 3 formulas in Table 1 are evaluated for stability under various storage conditions (three months at temperature up to 40°C, and relative humidity (RH) up to 75%). The lots are all manufactured at the same scale utilizing the same process and tested at about the same time using validated chemical analysis methods. Three-month data show that the presence of efinaconazole stabilizes tacrolimus in this base.

### Example 7: Comparison of combination lotion to tacrolimus-only lotion

The formula composition of the two products is provided in Table 10. Other than the difference in the drug substances (monad vs combination), the main difference is the composition of the oil phase solvents.

**Table 10: Quantitative Compositions of Tacrolimus Lotion and Combination Product**

| **Component** | **Concentration (% w/w)** | |
|---|---|---|
| | Tacrolimus Lotion | Formula C |
| Tacrolimus | 0.1 | 0.1 |
| Efinaconazole | 0 | 2.0 |
| Caprylic/Capric Triglycerides | 15 | 0 |
| Oleyl Alcohol | 10 | 0 |
| Diisopropyl Adipate | 0 | 20.0 |
| Diethyl Sebacate | 0 | 24.0 |
| Sodium Cetostearyl Sulfate | 1.0 | 1.0 |
| Mono- and Di-glyceride | 2.0 | 2.0 |
| Carbopol 981 (Carbomer Homopolymer Type A) | 0.2 | 0 |
| Carbopol 980 (Carbomer Homopolymer Type C) | 0 | 0.4 |
| Cetyl Alcohol | 1.0 | 1.5 |
| Stearyl Alcohol | 1.0 | 1.5 |
| Benzyl Alcohol | 1.0 | 1.0 |
| Propylene Glycol | 5.0 | 5.0 |
| Butylated Hydroxytoluene (BHT) | 0 | 0.1 |
| Citric Acid, Anhydrous | 0.05 | 0.1 |
| Edetate Disodium (EDTA) | 0 | 0.05 |
| Sodium Hydroxide | qs pH (5.7) | qs pH (5.7) |
| Purified water | qs to 100% | qs to 100% |

It is believed that tacrolimus and efinaconazole act in a mutually beneficial manner, each helping in retarding the degradation of the other drug substance.

## Claims

1. A topical pharmaceutical composition comprising a macrolide immunosuppressant and efinaconazole.

2. The pharmaceutical composition of claim 1 wherein the macrolide immunosuppressant is selected from the group consisting of pimecrolimus, sirolimus, and tacrolimus.

3. The pharmaceutical composition of claim 2 which is a topical cream formulation comprising 0.5 to 1.5 wt. %, preferably 0.9 wt.%, of pimecrolimus and 0.5 to 3 wt.% of efinaconazole.

4. The pharmaceutical composition of claim 2 which is a topical cream formulation comprising 0.1 to 1.0 wt. % of sirolimus and 0.5 to 3 wt.% of efinaconazole.

5. The pharmaceutical composition of claim 2 which is a topical cream formulation comprising 0.01 to 1 wt. % of tacrolimus and 0.5 to 3 wt.% of efinaconazole.

6. The pharmaceutical composition of any foregoing claim which is in the form of an emulsion comprising an oil phase, a water phase, one or more gelling agents, one or more surfactants, optionally one or more antioxidants, and optionally one or more chelators.

7. The pharmaceutical composition of any of claims 1 - 6 which is an emulsion comprising an oil phase, a water phase, one or more gelling agents, and one or more surfactants, wherein the oil phase comprises alkyl diesters of aliphatic dicarboxylic acids.

8. A topical composition comprising a combination of a macrolide immunosuppressant and efinaconazole for use in the treatment of an inflammatory skin HG:sch:djz condition, wherein the combination of a macrolide immunosuppressant and efinaconazole is administered to the affected area.

9. The topical composition comprising a combination of a macrolide immunosuppressant and efinaconazole for the use of claim 8, wherein an effective amount of the macrolide immunosuppressant and efinaconazole is administering to the affected area on at least a daily basis.

10. The topical composition comprising a combination of a macrolide immunosuppressant and efinaconazole for the use of claim 8, or 9 wherein the macrolide immunosuppressant and efinaconazole are in the form of a topical pharmaceutical composition according to any of claims 1 - 6.

11. The topical composition comprising a combination of a macrolide immunosuppressant and efinaconazole for the use of any one of claims 8 - 10 wherein the inflammatory skin condition is selected from seborrheic dermatitis, eczema, and psoriasis.

12. The topical composition comprising a combination of a macrolide immunosuppressant and efinaconazole for the use of any of claims 8 - 11 wherein the macrolide immunosuppressant is selected from the group consisting of pimecrolimus, sirolimus, and tacrolimus.

13. The topical composition comprising a combination of a macrolide immunosuppressant and efinaconazole for the use of any of claims 8 - 12 wherein the composition comprising the combination is an emulsion comprising an oil phase, a water phase, one or more gelling agents, and one or more surfactants, wherein the oil phase comprises alkyl diesters of aliphatic dicarboxylic acids.

14. A method of stabilizing a macrolide immunosuppressant in a topical composition, the method comprising admixing the macrolide immunosuppressant with efinaconazole.

## Patentansprüche

1. Eine topische pharmazeutische Zusammensetzung, umfassend ein Makrolid-Immunsuppressivum und Efinaconazol.

2. Die pharmazeutische Zusammensetzung nach Anspruch 1, wobei das Makrolid-Immunsuppressivum ausgewählt ist aus der Gruppe bestehend aus Pimecrolimus, Sirolimus und Tacrolimus.

3. Die pharmazeutische Zusammensetzung nach Anspruch 2, die eine topische Cremeformulierung umfassend 0,5 bis 1,5 Gew.-%, vorzugsweise 0,9 Gew.-%, Pimecrolimus und 0,5 bis 3 Gew.-% Efinaconazol ist.

4. Die pharmazeutische Zusammensetzung nach Anspruch 2, die eine topische Cremeformulierung umfassend 0,1 bis 1,0 Gew.-% Sirolimus und 0,5 bis 3 Gew.-% Efinaconazol ist.

5. Die pharmazeutische Zusammensetzung nach Anspruch 2, die eine topische Cremeformulierung umfassend 0,01 bis 1 Gew.-% Tacrolimus und 0,5 bis 3 Gew.-% Efinaconazol ist.

6. Die pharmazeutische Zusammensetzung nach einem der vorhergehenden Ansprüche, die in Form einer Emulsion, umfassend eine Ölphase, eine Wasserphase, ein oder mehrere Geliermittel, ein oder mehrere Tenside, gegebenenfalls ein oder mehrere Antioxidantien und gegebenenfalls einen oder mehrere Chelatbildner, vorliegt.

7. Die pharmazeutische Zusammensetzung nach einem der Ansprüche 1 bis 6, die eine Emulsion, umfassend eine Ölphase, eine Wasserphase, ein oder mehrere Geliermittel und ein oder mehrere Tenside, ist, wobei die Ölphase Alkyldiester von aliphatischen Dicarbonsäuren umfasst.

8. Eine topische Zusammensetzung umfassend eine Kombination aus einem Makrolid-Immunsuppressivum und Efinaconazol zur Verwendung bei der Behandlung einer entzündlichen Hauterkrankung, wobei die Kombination aus einem Makrolid-Immunsuppressivum und Efinaconazol auf die betroffene Stelle appliziert wird.

9. Die topische Zusammensetzung umfassend eine Kombination aus einem Makrolid-Immunsuppressivum und Efinaconazol zur Verwendung nach Anspruch 8, wobei eine wirksame Menge des Makrolid-Immunsuppressivums und Efinaconazol mindestens täglich auf die betroffene Stelle appliziert wird.

10. Die topische Zusammensetzung umfassend eine Kombination aus einem Makrolid-Immunsuppressivum und Efinaconazol zur Verwendung nach Anspruch 8 oder 9, wobei das Makrolid-Immunsuppressivum und Efinaconazol in Form einer topischen pharmazeutischen Zusammensetzung nach einem der Ansprüche 1 bis 6 vorliegen.

11. Die topische Zusammensetzung umfassend eine Kombination aus einem Makrolid-Immunsuppressivum und Efinaconazol zur Verwendung nach einem der Ansprüche 8 bis 10, wobei die entzündliche Hauterkrankung ausgewählt ist aus seborrhoischer Dermatitis, Ekzem und Psoriasis.

12. Die topische Zusammensetzung umfassend eine Kombination aus einem Makrolid-Immunsuppressivum und Efinaconazol zur Verwendung nach einem der Ansprüche 8 bis 11, wobei das Makrolid-Immunsuppressivum ausgewählt ist aus der Gruppe bestehend aus Pimecrolimus, Sirolimus und Tacrolimus.

13. Die topische Zusammensetzung umfassend eine Kombination aus einem Makrolid-Immunsuppressivum und Efinaconazol zur Verwendung nach einem der Ansprüche 8 bis 12, wobei die die Kombination umfassende Zusammensetzung eine Emulsion umfassend eine Ölphase, eine Wasserphase, ein oder mehrere Geliermittel und ein oder mehrere Tenside ist, wobei die Ölphase Alkyldiester von aliphatischen Dicarbonsäuren umfasst.

14. Ein Verfahren zur Stabilisierung eines Makrolid-Immunsuppressivums in einer topischen Zusammensetzung, wobei das Verfahren das Vermischen des Makrolid-Immunsuppressivums mit Efinaconazol umfasst.

## Revendications

1. Composition pharmaceutique topique comprenant un immunosuppresseur macrolide et de l'éfinaconazole.

2. Composition pharmaceutique selon la revendication 1 dans laquelle l'immunosuppresseur macrolide est choisi dans le groupe constitué par le pimécrolimus, le sirolimus et le tacrolimus.

3. Composition pharmaceutique selon la revendication 2 qui est une formulation de crème topique comprenant 0,5 à 1,5 % en poids, de préférence 0,9 % en poids de pimécrolimus et 0,5 à 3 % en poids d'éfinaconazole.

4. Composition pharmaceutique selon la revendication 2 qui est une formulation de crème topique comprenant 0,1 à 1,0 % en poids de sirolimus et 0,5 à 3 % en poids d'éfinaconazole.

5. Composition pharmaceutique selon la revendication 2 qui est une formulation de crème topique comprenant 0,01 à 1 % en poids de tacrolimus et 0,5 à 3 % en poids d'éfinaconazole.

6. Composition pharmaceutique selon l'une quelconque des revendications qui précèdent qui est sous la forme d'une émulsion comprenant une phase huileuse, une phase aqueuse, un ou plusieurs agents gélifiants, un ou plusieurs tensioactifs, éventuellement un ou plusieurs antioxydants et éventuellement un ou plusieurs chélateurs.

7. Composition pharmaceutique selon l'une quelconque des revendications 1 à 6 qui est une émulsion comprenant une phase huileuse, une phase aqueuse, un ou plusieurs agents gélifiants et un ou plusieurs tensioactifs la phase huileuse comprenant des alkyl diesters d'acides dicarboxyliques aliphatiques.

8. Composition pharmaceutique comprenant une combinaison d'un immunosuppresseur macrolide et d'éfinaconazole destinée à être utilisée dans le traitement d'un état de peau inflammatoire la combinaison d'un immunosuppresseur macrolide et d'éfinaconazole étant administrée à la zone affectée.

9. Composition topique comprenant une combinaison d'un immunosuppresseur macrolide et d'éfinaconazole destinée à être utilisée selon la revendication 8, une quantité efficace de l'immunosuppresseur macrolide et de l'éfinaconazole étant administrée à la zone affectée sur au moins une base quotidienne.

10. Composition topique comprenant une combinaison d'un immunosuppresseur macrolide et d'éfinaconazole destinée à être utilisée selon la revendication 8 ou 9 dans laquelle l'immunosuppresseur macrolide et l'éfinaconazole sont sous forme d'une composition pharmaceutique topique selon l'une quelconque des revendications 1 à 6.

11. Composition topique comprenant une combinaison d'un immunosuppresseur macrolide et d'éfinaconazole destinée à être utilisée selon l'une quelconque des revendications 8 à 10 dans laquelle l'état de peau inflammatoire est choisi parmi la dermatite séborrhéique, l'eczéma et le psoriasis.

12. Composition topique comprenant une combinaison d'un immunosuppresseur macrolide et d'éfinaconazole destinée à être utilisée selon l'une quelconque des revendications 8 à 11 dans laquelle l'immunosuppresseur macrolide est choisi dans le groupe constitué par le pimécrolimus, le sirolimus et le tacrolimus.

13. Composition topique comprenant une combinaison d'un immunosuppresseur macrolide et d'éfinaconazole destinée à être utilisée selon l'une quelconque des revendications 8 à 12 dans laquelle la composition comprenant la combinaison est une émulsion comprenant une phase huileuse, une phase aqueuse, un ou plusieurs agents gélifiants et un ou plusieurs tensioactifs, la phase huileuse comprenant des alkyl diesters d'acides dicarboxyliques aliphatiques.

14. Procédé de stabilisation d'un immunosuppresseur macrolide dans une composition topique, le procédé comprenant le mélange de l'immunosuppresseur macrolide avec l'éfinaconazole.
